## Europäisches Patentamt
## European Patent Office
## Office européen des brevets

(11) Veröffentlichungsnummer: **0 341 573**
**A2**

## EUROPÄISCHE PATENTANMELDUNG

(21) Anmeldenummer: 89108039.2

(22) Anmeldetag: 03.05.89

(51) Int. Cl.⁴ **B01D 13/00 , B01D 35/22**

(30) Priorität: 13.05.88 DE 3816334

(43) Veröffentlichungstag der Anmeldung:
15.11.89 Patentblatt 89/46

(84) Benannte Vertragsstaaten:
DE ES IT NL

(71) Anmelder: **Sartorius GmbH.**
**Weender Landstrasse 94-108**
**D-3400 Göttingen(DE)**

(72) Erfinder: **Schmidt, Hans-Weddo**
**Alte Uslaerstrasse 26 d**
**D-3414 Hardegsen(DE)**

(74) Vertreter: **Köhler, Rudolf**
**c/o Sartorius GmbH Weender Landstrasse**
**94-108**
**D-3400 Göttingen(DE)**

(54) Verfahren, Vorrichtung und Filtermodul zur Filtration von Flüssigkeiten im Cross-Flow-Betrieb.

(57) Bei einem Verfahren und einer Vorrichtung zur Verhinderung oder zum Abbau einer Filterdeckschicht bei der Filtration von Flüssigkeiten (F1,F1′) mit darin fein verteilten Bestandteilen im Cross-Flow-Betrieb, bei dem die zu behandelnde Flüssigkeit (F1,F1′) in einem engen Überströmungsspalt (SP) zwischen einer Filterfläche (PM) und einem Spaltbegrenzer (FM) hindurchgeführt wird, wird der Spaltbegrenzer (FM) durch Relativbewegung bezüglich der Filterfläche (PM) mit der sich aufbauenden Filterdeckschicht periodisch adhäsiv in Flächenkontakt gebracht und die Filterdeckschicht wird von der Filterfläche durch Reaktivierung des Überströmungsspaltes (SP) und Überströmung der Filterfläche und Spaltbegrenzerfläche spülend entfernt. Die bewegliche Wand ist durch eine randseitig dichtend in einem umgebenden Gehäuse (1 bis 7) eingespannte Folienmembran (FM) aus Kunststoff gebildet. Die Folienmembran (FM) ist auf der dem Überströmspalt (SP) abgewandten Seite durch eine Gasquelle (P/V) oder Flüssigkeitsquelle beaufschlagbar und die Filterfläche ist durch eine flexible permeable Membran (PM) gebildet. Durch die Zuordnung einer Steuereinrichtung (9...22) für die aufeinander abgestimmte Führung der Flüssigkeitsströme und Spaltverengung und Spaltvergrößerung ist ein quasi kontinuierlicher Betrieb möglich. Durch einen Rezirkulatzionskreislauf mit einem Vorratsbehälter insbesondere Fermenter (20) ist auch eine Probeentnahme von aktuellem Fermenterfiltrat (F2) möglich.

Fig. 1

Verfahren, Vorrichtung und Filtermodul zur Filtration von Flüssigkeiten im Cross-Flow-Betrieb

Die Erfindung betrifft ein Verfahren, eine Vorrichtung und ein Filtermodul zum Abbau bzw. zur Verhinderung der Bildung einer Filterdeckschicht bei der Filtration von Flüssigkeiten mit darin fein verteilten Bestandteilen im Cross-Flow-Betrieb, bei dem die zu behandelnde Flüssigkeit in einem engen Überströmspalt zwischen einer Filterfläche und einem flüssigkeitsundurchlässigen Spaltbegrenzer hindurchgeführt wird.

Bei einer bekannten Vorrichtung für die Ultrafiltration (DE-OS 26 53 875) wird vorgeschlagen, zur Vermeidung der Deckschichtbildung die Spalthöhe möglichst klein zu halten, um somit auch die sich bildende Filterdeckschicht möglichst klein zu halten. Dies soll konstruktiv dadurch erreicht werden, daß zwischen zwei Filterplatten mit darauf liegendem Filtermedium ein elastisches undurchlässiges Druckkissen angeordnet ist, welches jeweils die eine Wand der spaltförmigen Filterkammer bildet. Das Druckkissen soll dabei einen Druckausgleichskörper bilden, der sich während des Durchströmens der Flüssigkeit entsprechend den Druckverhältnissen an seiner Oberfläche verformt. Dadurch soll über die Flüssigkeit überall der gleiche lokale Strömungswiderstand erzeugt werden.

Es hat sich jedoch in der Praxis herausgestellt, daß sich in bestimmten Bereichen bevorzugte Strömungspfade ausbilden, und die übermäßige Bildung einer Deckschicht nur partiell begrenzt werden kann.

Bei nach dem Cross-Flow-Verfahren betriebenen Filterelementen ist es auch üblich (DE-OS 34 41 249), eine sich auf der mikroporösen Filtermembran bildende Filterdeckschicht teilweise dadurch zu entfernen, daß das Filterelement von der Filtratseite her mit Filtrat oder einem Spülmittel beaufschlagt wird und die Filterdeckschicht anschließend weggespült wird. Dies führt dazu, daß während des eigentlichen Filtrationsbetriebes ständig zwischen Filtration und Rückspülung mit dem bereits gewonnenen Filtrat gewechselt werden muß und sich der Rückspülvorgang an sich nur bei einer Generalreinigung mit neutralem Spülmittel rentiert. In beiden Fällen der Rückspülung wird das Filterelement also auch entgegen der eigentlichen Filtrationsrichtung gestresst.

Es ist auch bekannt (DE-OS 34 11 471), bei Schlauchfiltern die aufgebaute Deckschicht dadurch abzubauen, daß die Schlauchfilter den Filtratraum frei durchsetzen und deren Querschnitt bei filtratseitigem Überdruck reversibel flexibel und unregelmäßig kollabiert. Besonders durch diese pulsartige Kollabierung werden die Filterelemente stark gestresst, was zu Rupturen führen kann.

Die Filtration nach dem Cross-Flow-Verfahren gewinnt in der Getränkeindustrie immer mehr Bedeutung. Die zu filtrierenden Flüssigkeiten sind mehr oder weniger stark mit feinverteilten Bestandteilen belastet. Bei der Filtration von ungeschönten Weinen im Cross-Flow-Verfahren erfolgt eine sehr schnelle und starke Belegung der Filtermembranen mit einer Filterdeckschicht auch wenn versucht wird, dies mit hohen Strömungsgeschwindigkeiten zu verzögern.

Ähnliche Probleme treten auf, wenn eine Probeentnahme aus einer zirkulierenden Fermenterbrühe erfolgen soll. Die über ein Filterelement nach dem Cross-Flow-Prinzip geführte Fermenterbrühe belegt die Filtermembran sehr schnell mit einer Filterdeckschicht. Das auf der Filtratseite abgezogene Fermenterfiltrat gibt damit häufig nicht den aktuellen Zustand der Fermenterbrühe wieder, da die im Filtrat enthaltenen Zellen aus der Zellkultur nicht aus der aktuellen Fermenterbrühe stammen, die an der Deckschicht vorbeigeführt ist, sondern alte Zellen oder geschädigte Zellen sind, die sich bereits in der Filterdeckschicht befinden.

Der Erfindung liegt daher die Aufgabe zugrunde, mit einfachen Mitteln auf eine für das Filterelement schonende Weise den Abbau bzw. die Verhinderung einer Filterdeckschicht beim Betrieb einer Filteranlage nach dem Cross-Flow-Verfahren zu ermöglichen.

Diese Aufgabe wird erfindungsgemäß dadurch erreicht, daß der Spaltbegrenzer durch Relativbewegung bezüglich der Filterfläche mit der sich aufbauenden Filterdeckschicht periodisch adhäsiv in Flächenkontakt gebracht wird und die Filterdeckschicht von der Filterfläche durch Reaktivierung des Überströmspaltes und Überströmung der Filterfläche und Spaltbegrenzerfläche spülend entfernt wird. Der von außen zugeführte Flüssigkeitsstrom an zu filtrierender Flüssigkeit kann dabei im Überströmspalt periodisch unterbrochen werden oder aber der Flüssigkeitsstrom wird von außen her während der Phase der Spaltverengung verlangsamt. In der Phase der Spaltverengung beziehungsweise der erneuten Spalterweiterung kommt es nach dem Venturirohr-Prinzip zu einer Erhöhung der Strömungsgeschwindigkeit im Spalt und bei der adhäsiven flächigen Kontaktierung der Filterdeckschicht durch die relativ glatte und undurchlässige Oberfläche des Spaltbegrenzers wird die Deckschicht von dem porösen Filterelement abgelöst, so daß beide Effekte zu dem erwünschten Abspülvorgang führen. Die Ablösung der Filterdeckschicht ist um so intensiver, je feinporiger die der Filterdeckschicht zugewandte Oberfläche des Filterelementes ist, so daß insbesondere bei der Verwendung von Ultrafiltrationsmembranen und Mikrofiltrationsmembranen ein besonders effektiver Abbau der Filterdeckschicht bzw. deren Verhinderung erreicht werden kann.

Der Spaltbegrenzer ist vorzugsweise als fluiddichte Folienmembran gebildet, die auf der dem Filterelement abgewandten Seite durch Flüssigkeitszufuhr in eine Kammer oder durch Druckgas oder Vakuum zur Spaltbildung etwa in der Größenordnung von 0 - 1000 μm verstellt werden kann. Bei einer pulsartigen Durchführung der vorstehend beschriebenen Bewegungsabläufe der Membran und Strömungsführung der zu behandelnden Flüssigkeit wird der Abbau der Filterdeckschicht durch Turbulenzen noch unterstützt.

Filterelemente können sowohl poröse feste Filterelemente z.B. Keramik- oder Glasfilter sein als auch membranartig ausgebildete flexible Filter, mikroporös oder als relativ dichte Filterfolien für die Pervaporation.

Der undurchlässige Spaltbegrenzer kann durch eine bewegliche Wand eines das Filterelement umschließenden Gehäuses gebildet sein. Die Wand kann eine kolbenförmig bewegliche Platte vorzugsweise jedoch eine randseitig dichtend in einem umgebenden Gehäuse eingespannte Folienmembran aus Kunststoff sein.

Über eine der Filtrationseinrichtung zugeordnete Steuereinrichtung unter Verwendung von Flüssigkeitspumpen, Gaspumpen und Ventilen ist ein quasi kontinuierlicher Filtrationsbetrieb möglich, ohne daß bereits gewonnenes Filtrat wieder dazu benutzt werden muß, von der Filtratseite her durch die Filtermembran hindurch in den Retentatkreislauf eingespeist zu werden.

Der Erfindungsgedanke ist in mehreren Ausführungsbeispielen anhand der beiliegenden Zeichnung näher erläutert. Dabei zeigt:

Fig. 1 bis Fig. 4 schematisch einen Querschnitt durch eine Filtervorrichtung, angeordnet in einem Fermenterkreislauf als Entnahmegerät in verschiedenen Filtrationsphasen,

Fig. 5 einen schematisch vereinfachten Horizontalschnitt durch den mittleren Gehäuseteil des Filtergerätes gemäß Fig. 1 bis 4,

Fig. 6 schematisch einen Vertikalschnitt durch eine rechteckige Ausführungsform nach der Linie 6-6 in Fig. 7,

Fig. 7 einen Horizontalschnitt nach der Linie 7-7 in Fig. 6,

Fig. 8 eine Draufsicht auf die Filtratplatte für ein rundes stapelförmiges Filtermodul,

Fig.9 + 10 eine Draufsicht auf Abstandshalteplatten,

Fig. 11 einen perspektivischen Vertikalschnitt durch das stapelförmige Filtermodul nach der Schnittlinie 11-11 in Fig. 8 in Explosionsdarstellung und

Fig. 12 eine Explosivdarstellung ähnlich der Fig. 11 nach der Linie 12-12 in Fig. 8.

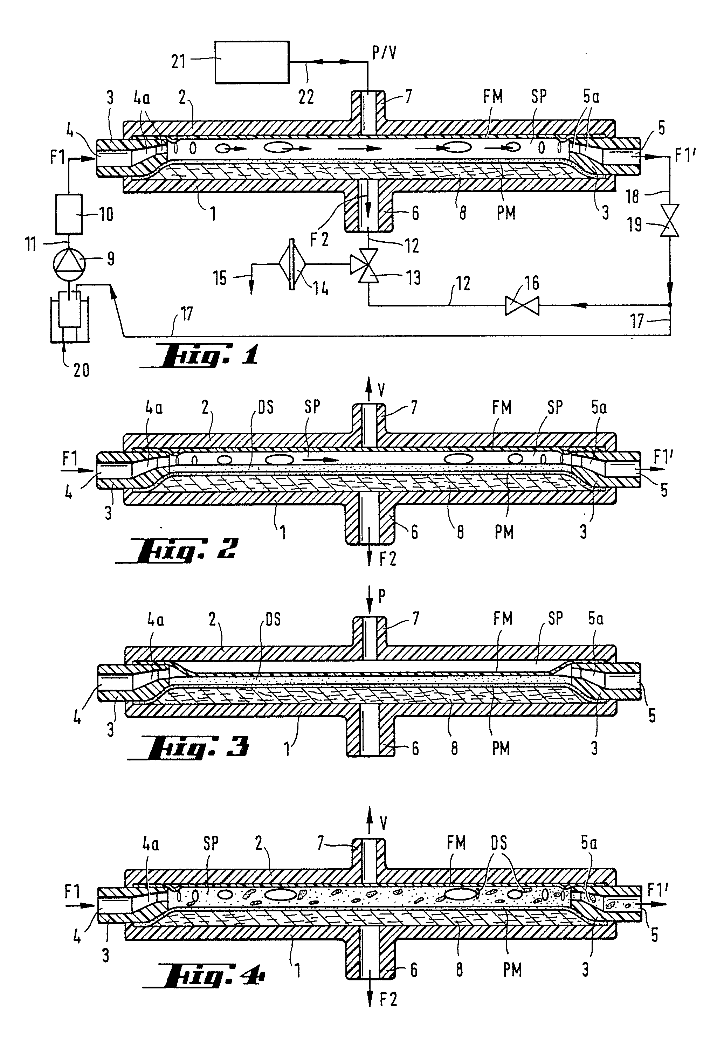

Die Filtervorrichtung gemäß Fig. 1 bis 4 besteht aus dem unteren Gehäuseteil 1 mit einem Anschluß 6 für Filtrat, dem mittleren Gehäuseteil 3 mit diametral gegenüberliegenden Anschlüssen 4,5 für die zu behandelnde Flüssigkeit F1,F1' und dem oberen Gehäuseteil 2 mit einem Anschluß 7 für Druckmittel P in Form von Gas oder Flüssigkeit oder Vakuum V. Zwischen dem oberen Gehäuseteil 2 und dem mittleren Gehäuseteil 3 ist randseitig eine Flüssigkeitsdichte, glatte Folienmembran FM dichtend eingespannt, deren rückwärtige Seite über den Anschluß 7 mit Fluid beaufschlagbar und parallel zu der eigentlichen Filterfläche in Form einer porösen Membran PM beweglich ist. Diese poröse Membran PM ist zwischen dem mittleren Gehäuseteil 3 und dem unteren Gehäuseteil 1 angeordnet und auf ihrer Unterseite, d.h. Filtratseite durch eine flächige, poröse Drainageschicht 8 abgestützt. Sowohl die Folienmembran FM als auch die poröse Membran PM sind in üblicher Weise über umlaufende Dichtungsringe oder durch Verschweißung gegenüber den Gehäuseteilen 1 bis 3 zur Bildung getrennter Fluidkammern abgedichtet. Zwischen beiden Membranen FM und PM ist ein relativ enger Überströmspalt SP gebildet, der erfindungsgemäß durch Beaufschlagung der Folienmembran FM in der Größenordnung von 0 - 1000 μm während des Filtrationsbetriebes einstellbar ist.

Die Filtervorrichtung ist Teil hier einer Probeentnahmevorrichtung für einen Fermenter 20. Zu diesem Zweck ist der Anschluß 4 für die zu behandelnde Flüssigkeit F1, hier Fermenterbrühe über die Leitung 11, das Rückschlagventil 10 und die Pumpe 9 an den Fermenter 20 angeschlossen. Der Anschluß 5 für die nicht aufkonzentrierte oder aufkonzentrierte Fermenterbrühe F1' ist über die Leitung 18, das Ventil 19 und die Leitung 17 im Kreislauf ebenfalls an den Fermenter 20 angeschlossen. Der Anschluß 6 für das Filtrat F2, ein 3-Wege-Ventil 13, eine Leitung 12 und ein Ventil 16 sind ebenfalls an die zum Fermenter 20 führende Leitung 17 angeschlossen. Über das 3-Wege-Ventil 13 ist über eine Bypass-Leitung 15 mit dazwischen angeordneten Sterilfilter 14 eine Probeentnahmestelle für Fermenterfiltrat gebildet. Ein Druck-Vakuumgeber 21 versorgt über die Leitung 22 den Anschluß 7 entweder mit flüssigem oder gasförmigem Druckmittel P/V.

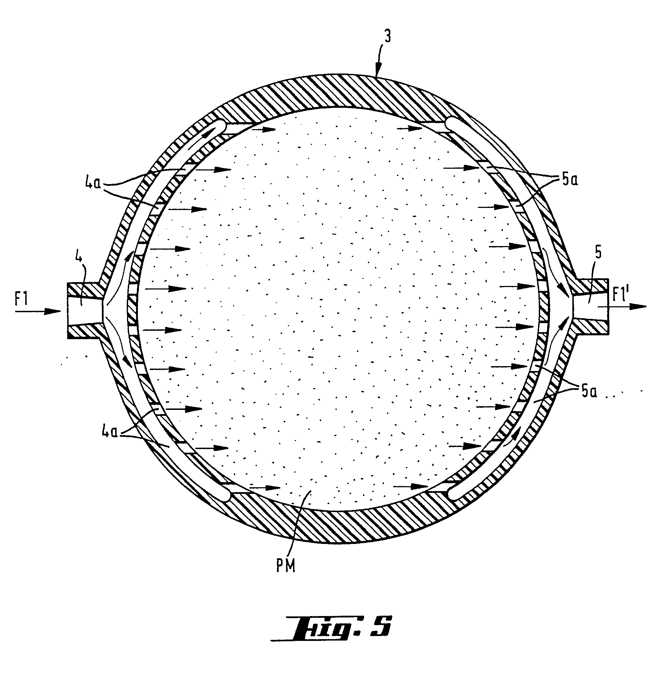

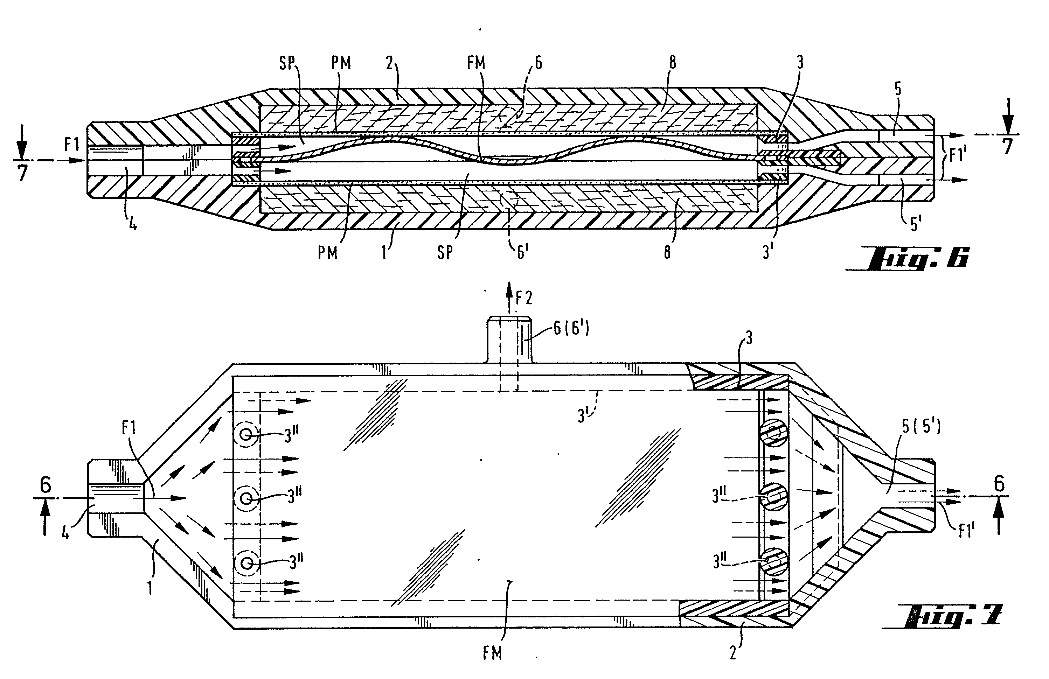

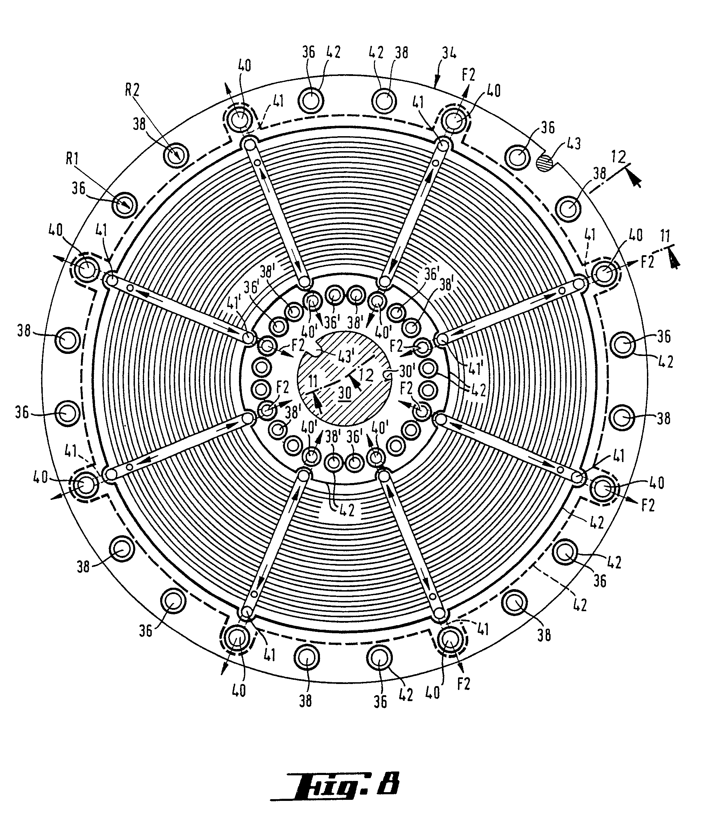

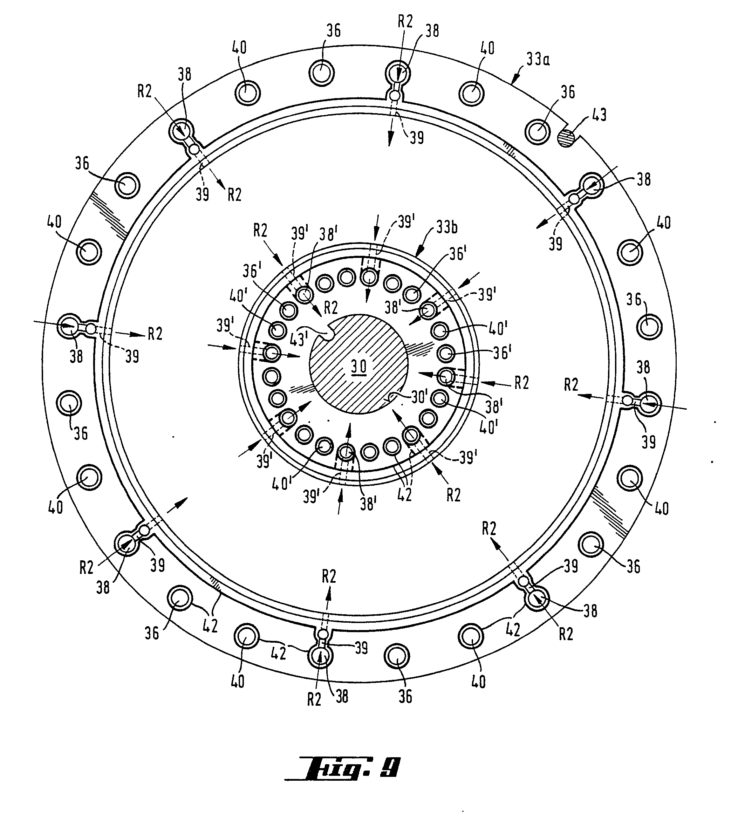

Sobald sich während des Filtrationsbetriebes oder auch bei der reinen Überströmung ohne Abzug von Filtrat in dem Überströmspalt SP in der Einstellung gemäß Fig. 1 oder auch in einer dazwischenliegenden engeren Einstellung etwa gemäß Fig. 3 eine Filterdeckschicht DS aufbaut, wird der Überströmspalt SP gemäß Fig. 2 periodisch soweit durch Beaufschlagung der Folienmembran FM verengt, daß diese mit der Oberfläche der Filterdeckschicht DS adhäsiv flächig gemäß Fig. 3 in Kontakt kommt. Die Adhäsionskräfte zwischen der relativ glatten Folienmembran FM und der Filterdeckschicht DS führen bei anschließender Erweiterung des Überströmspaltes SP zu einem Abriß der Grenzfläche der Filterdeckschicht DS und damit Auflösung der Querschnittsstruktur, so daß die durch den verengten Überströmspalt SP nach dem Prinzip eines Venturi-Kanals mit erhöhter Strömungsgeschwindigkeit im Überströmspalt SP fließende Flüssigkeit F1 aufgelöste Filterdeckschicht DS gemäß Fig. 4 in den Fermenterkreislauf zurückgespült wird. Die bei der spülenden Überströmung eintretende Bewegung der flexiblen Folienmembran FM unterstützt die Auflösung der Filterdeckschicht DS, so daß diese je nach Porengröße der porösen Membran PM bzw. eines starren Filtermediums bis auf Rückstände in den Poren entfernt wird.

Die drainierende Filterunterstützung 8 ist zweckmäßig in Form von Rillen sehr eng gehalten bzw. durch ein flächiges Stützvlies gebildet, so daß auch auf der porösen Membran PM keine wellenförmigen Erhebungen und Vertiefungen gebildet werden und somit der Überströmspalt SP durch zwei parallel zueinander verlaufende Begrenzungen gebildet ist.

Durch die pulsierende Beaufschlagung der Folienmembran FM in Abstimmung mit der Strömungsführung der Flüssigkeit F1, F1$'$ und F2 läßt sich die poröse Membran PM frei von einer Filterdeckschicht halten, so daß bei einer Probeentnahme aus der Entnahmestelle 15 jeweils das aktuelle Fermenterfiltrat entnommen werden kann. Zu diesem Zweck wird lediglich das Ventil 16 in der Leitung 12 geschlossen. Die Entnahme erfolgt also ohne daß empfindliche Zellkulturen eine schädigende Filterdeckschicht DS durchdringen müssen.

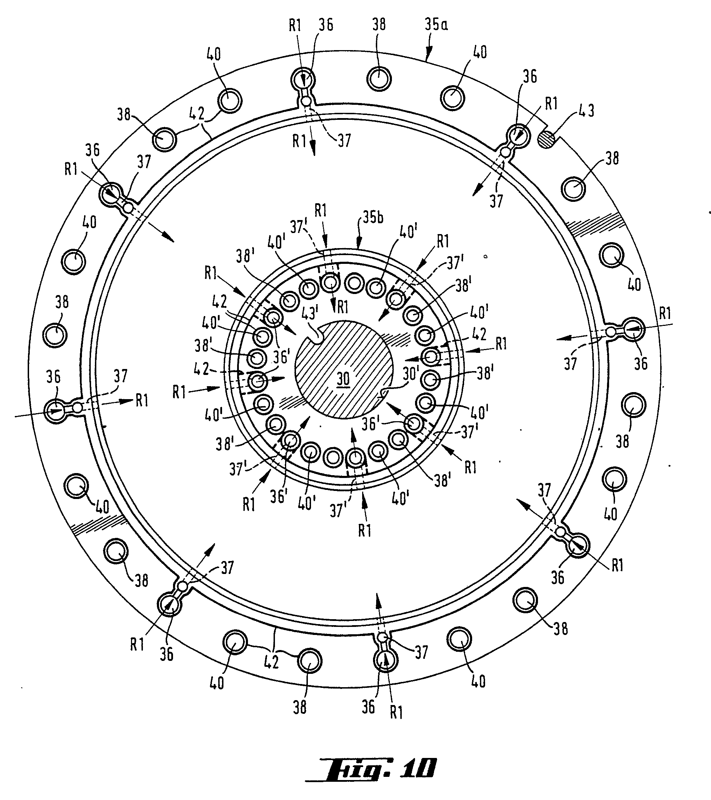

Fig. 5 zeigt schematisch vereinfacht einen Schnitt durch das mittlere Gehäuseteil 3 für das im wesentlichen kreisrunde Filtergerät. Die Anschlüsse 4,5 gehen dabei in eine Vielzahl von Verteilerkanälen 4a bzw. Sammelkanälen 5a über.

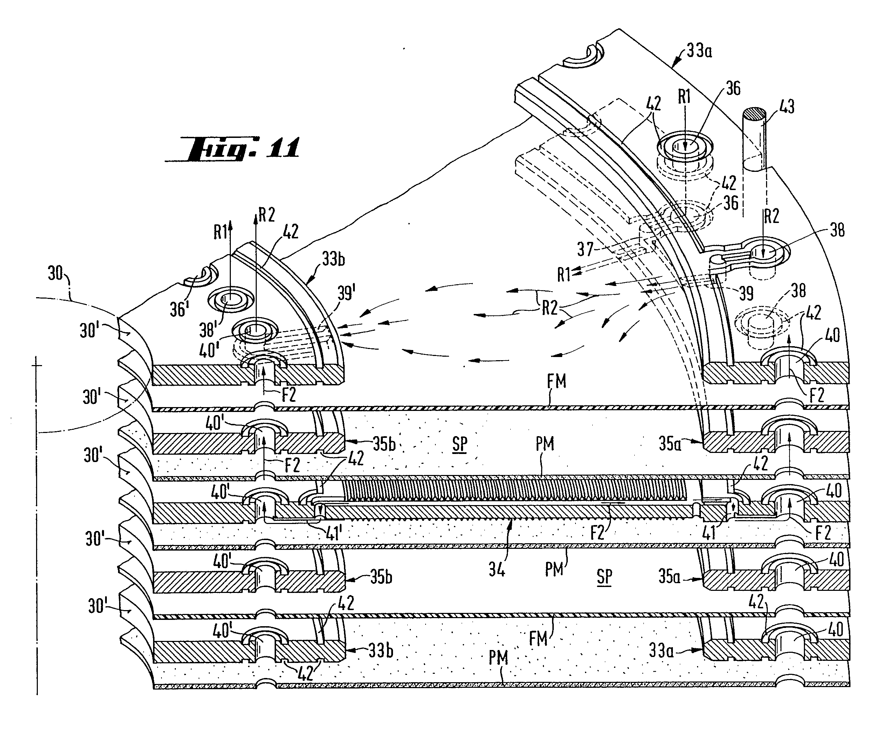

In der Ausführungsform nach Fig. 6 und 7 bildet die bewegliche Wand einen den Überströmspalt in zwei Teilspalte aufteilenden Strömungsteiler in Form einer beidseitig überströmbaren Folienmembran FM, welche zwischen den Überströmspalt limitierenden Wänden (poröse Membranen PM) beweglich ist. Die Folienmembran FM ist dabei umlaufend zwischen Gehäuseteilen 1,2,3,3$'$ eingespannt, wobei die Überströmung in den beiden Teilspalten parallel oder bei einem zusätzlichen Anschluß 4 im Gegenstrom erfolgen kann. Die Folienmembran FM pulsiert aufgrund ihrer Übergröße bzw. Flexibilität und gegebenenfalls Dehnbarkeit maximal zwi schen den begrenzenden Spaltwänden PM. Die Folienmembran FM kann Z.B. aus Silikon bestehen und nach Art einer Lochkarte durch Zapfen 3$''$ im Gehäusemittelteil 3,3$'$ am Einlaß und Auslaß 4,5 fixiert und an den anderen Seitenrändern eingespannt sein.

Ist die Überströmkammer rechteckig ausgebildet, so genügt es, wenn die Folienmembran FM an zwei gegenüberliegenden Seiten als Strömungsteiler im Gehäuse 1 bis 3 eingespannt ist, wobei die Folienlänge zwischen den Einspannstellen etwas größer gehalten ist als die Distanz zwischen den Einspannstellen, so daß auch hier die Folie pulsierend sich zwischen Grenzlagen bewegen kann. Durch Absperr- oder Drosselventile an den Anschlüssen 4,5.5$'$,6,6$'$ lassen sich die Lagen und Positionen der Folienmembran FM zwischen ihren Begrenzungswänden PM beeinflussen.

Es ist auch möglich, in einer rechteckig ausgebildeten Überströmkammer eine rechteckige Folie nur an dem der Anströmseite zugewandten Ende als Strömungsteiler im Gehäuse einzuspannen. Die mit ihrem anderen Ende frei beweglich im Überströmspalt fahnenartig flatternde Folie verhindert damit eine Deckschichtbildung.

Bei der Ausführungsform nach Fig. 8 bis 12 werden kreisrunde Einzelelemente auf einer unteren Endplatte um einen damit verbundenen zentralen Spannbolzen 30 gestapelt und von einer weiteren oberen Endplatte mit Fluidanschlüssen abgedeckt, die sich auf dem Spannbolzen 30 führt und durch Spannmittel gegen den Stapel aus Einzelelementen gepreßt wird, wobei am Spannbolzen 30 und/oder an der Peripherie der kreisrunden Einzelelemente Fixierungselemente 43$'$ und 43 vorgesehen sind, welche dafür sorgen, daß die die Einzelelemente durchsetzenden Durchbrechungen im Stapel fluchten und in der richtigen Reihenfolge in der Umfangsrichtung korrespondieren. Bei der nachfolgend beschriebe nen Ausführungsform ist die Folienmembran FM zwischen zwei porösen Membranen PM unter Zwischenlage von Abstandshalterrahmen 33a,33b bzw. 35a.35b angeordnet und die Druckbeaufschlagung der Folienmembran FM in Richtung der einen oder anderen porösen Membran PM erfolgt mit Hilfe der zu behandelnden Flüssigkeit, so daß diese zur besseren Unterscheidung mit Retentat R1 und Retentat R2 bezeichnet wird.

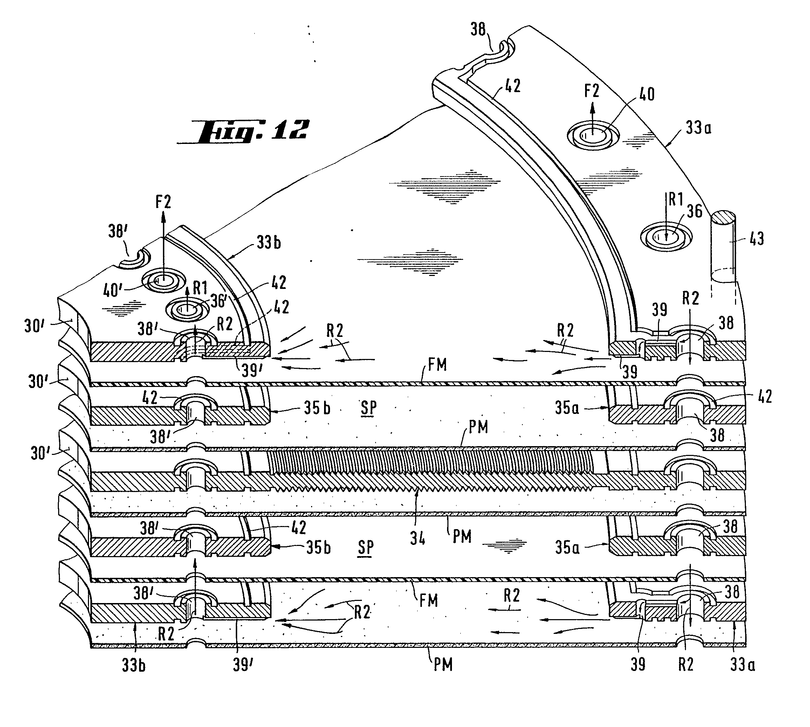

Die Filtratstützplatte 34 gemäß Fig. 8 und zu sehen im Randdetailschnitt gemäß Fig. 9 und 10 hat über den Umfang verteilt angeordnete Durchbrechungen 36 am Außenrand und Durchbrechungen 36$'$ am Innenrand für die Ein- und Ausführung von Retentat R1, entsprechende Durchbrechungen 38,38$'$ zur Ein- und Ausführung von Retentat R2, Durchbrechungen 40,40$'$ und Verbindungskanäle 41,41$'$ zur Abführung von Filtrat F2, wobei die Durchbrechungen durch Dichtungselemente 42 ganz oder teilweise umschlossen sind. Die Filtratstützplatte 34 weist eine Vielzahl von konzentrisch angeordneten Rippen und Kanäle sowie drainierende Radialkanäle auf, so daß eine ebene Abstützfläche für die poröse Membran PM gemäß Fig. 9 bis 12 gebildet ist.

Die Filtratstützplatte 34 ist gemäß Fig. 11 und 12 beidseitig durch eine poröse Membran PM belegt, welche bis an den äußeren Rand und an den inneren Rand 30' (Fig. 8) reicht.

Die beiden porösen Membranen PM sind durch innere (35b) und äußere (35a) Abstandshalterrahmen von einer entsprechend bemessenen Folienmembran FM getrennt und letztere von einer weiteren benachbarten porösen Membran PM durch einen weiteren Typ von inneren (33b) und äußeren (33a) Abstandshalterrahmen mit Verbindungskanälen 39,39' an den Durchbrechungen 38,38' für die Führung von Retentat R2. Der erste Typ von Abstandshalterrahmen 35a,35b hat ebenfalls Verbindungskanäle 37,37' für die Führung von Retentat R1 in den Durchbrechungen 36,36'. Die Durchbrechungen 36,36',38,38' und 40,40' gehen durch sämtliche Elemente also Abstandshalterrahmen 33,35,33a,33b,35a,35b Folienmembranen FM, Filtratstützplatte 34 und poröser Membran PM und gegebenenfalls durch mindestens eine Endplatte hindurch.

Bei der dargestellten Ausführungsform handelt es sich bei den Abstützplatten 34 und Abstandshalterrahmen 33a,33b, 35a,35b entweder um dünne Metallplatten oder um dünne Kunststoffplatten. Bei ersterem Fall sind die Dichtungselemente 42 einschließlich der übrigen Profilierung in Form von Rillen, Verbindungskanälen und Durchbrechungen im Ätzverfahren hergestellt. Durch Wahl anderer Fertigungstechniken läßt sich die Geometrie der Strömungsführung z.B. mittels einfacher Radialkanälen und Durchlässen vereinfachen. Sowohl die Folienmembran FM als auch die poröse Membran PM sind durch die rinnenförmigen Dichtungselemente 42 durch Preßdruck und Quellung in diesem Bereich abgedichtet.

Das bei der schematischen Ausführungsform gemäß Fig. 1 bis 4 beschriebene Verfahren zur Verhinderung oder zum Abbau einer Filterdeckschicht erfolgt dadurch, daß die gleichzeitige Einströmung von Retentat R1 und R2 in die Durchbrüche 36 und 38 abwechselnd periodisch reduziert wird, so daß sich z.B. die in Fig. 11 und 12 zu oberst gezeichnete Folienmembran FM bei Druckerhöhung von R2 und Druckreduzierung von R1 nach unten bewegt und die poröse Membran PM bzw. die Filterdeckschicht kontaktiert. Durch Druckerhöhung bei R1 und Druckreduzierung bei R2 bewegt sich dieselbe Folienmembran FM in Richtung auf eine in Fig. 11 und 12 nicht mehr dargestellte darüberliegende poröse Membran mit entsprechender Filterstützplatte 34. Dadurch wird die Filterdeckschicht von der ersten porösen Membran PM abgelöst und weggespült währenddessen die filtrierende Überströmung der darüberliegenden porösen Membran PM durch den Retentatstrom R2 und über die Durchbrechungen 38,38' und Verbindungskanäle 39,39' erfolgt.

Durch eine entsprechende Pulssteuerung ist ein quasi kontinuierlicher Filtrationsbetrieb möglich.

Selbstverständlich ist es möglich, anstelle der Druckbeaufschlagung durch das Retentat R1 bzw. R2 selbst ein separates Druckmittel in Form einer Flüssigkeit oder eines Gases einzuspeisen. Hierzu ist es nur notwendig, daß jeweils zwei Folienmembranen FM durch einen inneren und äußeren Abstandshalterrahmen 35a,35b bzw. 33a,33b voneinander in Abstand gehalten sind und diese speziellen Strömungskammern gegenüber den Retentatkammern und Filtratkammern abgedichtet sind. Die Steuerung der Fluidströme erfolgt über Regelgeräte und Steuerventile.

Das zwischen zwei Folienmembranen FM eingeführte Fluid für eine pulsierende Bewegung der beiden Folienmembranen kann auch ein Wärmetauschermedium sein, um die zu filtrierenden Flüssigkeiten entsprechend den Bedürfnissen temperieren zu können.

Das vorbeschriebene Verfahren hat den Vorteil, daß die Deckschicht bzw. der Filterkuchen schnellstmöglich abgebaut oder die·Entstehung direkt unterbunden werden kann, da dieser keiner Alterung·mehr unterliegen und dadurch nicht mehr verhärten kann. Besonders vorteilhaft ist es, daß die vorgenannte Filtrationseinheit mit kleiner Pumpleistung auskommt und die in Anspruch genommene Energie direkt and der Filtermembrane. zur Wirkung kommt. Ein ständig wechselnder Stress der Filterelemente durch abwechselnden Filtrationsbetrieb und Rückspülung, wie er bei bekannten Methoden zum Deckschichtabbau auftritt, wird nach der Erfindung vermieden.

## Ansprüche

1. Verfahren zur Verhinderung oder zum Abbau einer Filterdeckschicht bei Filtration von Flüssigkeiten mit darin fein verteilten Bestandteilen im Cross-Flow-Betrieb, bei dem die zu behandelnde Flüssigkeit in einem engen Überströmungsspalt zwischen einer Filterfläche und einem Spaltbegrenzer hindurchgeführt wird, dadurch gekennzeichnet, daß der Spaltbegrenzer (FM) durch Relativbewegung bezüglich der Filterfläche (PM) mit der sich aufbauenden Filterdeckschicht (DS) bzw. mit der Filterfläche (PM) periodisch adhäsiv in Flächenkontakt gebracht wird und die Filterdeckschicht (DS) von der Filterfläche durch Reaktivierung des Überströmungsspaltes (SP) und Überströmung der Filterfläche und Spaltbegrenzerfläche spülend entfernt wird.

2. Verfahren nach Anspruch 1, dadurch gekennzeichnet, daß der Flüssigkeitsstrom im Überströmspalt (SP) periodisch unterbrochen wird.

3. Verfahren nach Anspruch 1, dadurch gekennzeichnet, daß der Flüssigkeitsstrom im Überströmspalt (SP) während der Phase der Spaltverengung verlangsamt wird.

4. Vorrichtung zur Verhinderung oder zum Abbau einer Filterdeckschicht bei Filtration von Flüssigkeiten mit darin fein verteilten Bestandteilen im Cross-Flow-Betrieb, bei dem die zu behandelnde Flüssigkeit (F1) in einem engen Überströmspalt (SP) zwischen einer Filterfläche und einem parallelen flüssigkeitsundurchlässigen Spaltbegrenzer hindurchführbar ist, dadurch gekennzeichnet, daß der Spaltbegrenzer (FM) und die zugewandte Filterfläche relativ gegeneinander bis zur Kontaktierung mit einer sich im Überströmspalt (SP) auf der Filterfläche (PM) aufbauenden Filterdeckschicht (DS) bzw. bis zur Kontaktierung der Filterfläche (PM) bewegbar und wieder voneinander entfernbar sind.

5. Vorrichtung nach Anspruch 4, dadurch gekennzeichnet, daß der Spaltbegrenzer durch eine bewegliche Wand eines das Filterelement umschließenden Gehäuses gebildet ist.

6. Vorrichtung nach Anspruch 4, dadurch gekennzeichnet, daß die bewegliche Wand durch eine kolbenförmig bewegliche Platte gebildet ist.

7. Vorrichtung nach Anspruch 4, dadurch gekennzeichnet, daß die bewegliche Wand durch eine randseitig dichtend in einem umgebenden Gehäuse eingespannte Folienmembran (FM) aus Kunststoff gebildet ist.

8. Vorrichtung nach Anspruch 4, dadurch gekennzeichnet, daß die Folienmembran (FM) auf der dem Überströmspalt (SP) abgewandten Seite durch eine Gasquelle (P V) oder Flüssigkeitsquelle beaufschlagbar ist.

9. Vorrichtung nach Anspruch 4, dadurch gekennzeichnet, daß die Filterfläche durch eine flexible poröse Membran (PM) gebildet ist.

10. Vorrichtung nach Anspruch 4, dadurch gekennzeichnet, daß die poröse Membran (PM) auf der Filtratseite flächig drainierend unterstützt ist.

11. Vorrichtung nach Anspruch 4, dadurch gekennzeichnet, daß die poröse Membran (PM) eine Ultrafiltrationsmembran ist.

12. Vorrichtung nach Anspruch 4, dadurch gekennzeichnet, daß die poröse Membran (PM) eine Mikrofiltrationsmembran ist.

13. Vorrichtung nach Anspruch 4, dadurch gekennzeichnet, daß die Filterfläche durch eine permeable Pervaporationsmembran gebildet ist.

14. Filtermodul zur Filtration von Flüssigkeiten mit darin fein verteilten Bestandteilen im Cross-Flow-Betrieb mit in einem umgebenden Gehäuse mit Anschlüssen (4,5,6) zwischen Gehäuseteilen (1,2,3) randseitig dichtend eingeschlossener permeabler Filtermembran (PM), die auf einer Seite eine Überströmfläche für die zu behandelnde Flüssigkeit (F1) und auf der anderen Seite eine drainierte Fläche zur Abführung des Filtrats (F2) bildet, wobei die Überströmfläche der permeablen Membran (PM) etwa parallel dazu durch einen Spaltbegrenzer überdeckt ist, dadurch gekennzeichnet, daß der Spaltbegrenzer durch eine randseitig an Gehäusenteilen (2,3) gehaltene flexible Folienmembran (FM) gebildet ist, welche auf der der Überströmfläche abgewandten Seite durch Druckmittel in ihrem Abstand zur Überströmfläche der permeablen Membran begrenzt mobil ist.

15. Filtermodul nach Anspruch 14, dadurch gekennzeichnet, daß zwischen randseitig zwischen Gehäuseteilen eingespannten permeablen Membranen (PM) und Folienmembranen (FM) umlaufende, den Überströmspalt (SP) limitierende Abstandshalterrahmen (33a,33b,35a,35b) angeordnet sind, welche Strömungskanäle (36...40 und 36',38', 40') für die Strömungsführung aufweisen.

16. Filtermodul nach Anspruch 14 und 15, dadurch gekennzeichnet, daß zwischen zwei permeablen Membranen (PM) eine drainierende Stützplatte (34) mit Strömungskanälen (36, 38,40,41,36',38',40',41') angeordnet ist.

17. Filtermodul nach Anspruch 14 bis 16, dadurch gekennzeichnet, daß zwischen zwei Folienmembranen (FM) umlaufende, den Überströmspalt (SP) limitierende Abstandshalterrahmen (33a,33b,35a,35b) angeordnet sind.

18. Filtermodul nach Anspruch 14 bis 17, dadurch gekennzeichnet, daß mehrere permeablen Membranen (PM), Folienmembranen (FM), Abstandshalterrahmen (33a,33b,35a,35b) und drainierende Filterstützplatten (34) aufeinandergestapelt zu einer Strömungskammern bildenden Einheit abdichtend verbunden und ihre Strömungskanäle (36,38, 40,41,36',38',40' 41') über Leitungen mit Fluid beaufschlagbar sind.

19. Filtermodul nach Anspruch 14 bis 18, dadurch gekennzeichnet, daß die die Strömungskammern bildende stapelförmige Einheit um einen zentralen Spannbolzen (30) angeordnet ist und die stapelförmige Einheit an beiden Enden durch Endplatten abgedeckt sind, von denen mindestens eine auf dem Spannbolzen (30) beweglich geführt ist und von denen mindestens eine die Leitungsanschlüsse für die Versorgung der Fluidkammern aufweist.

20. Filtermodul nach Anspruch 8 und 4, dadurch gekennzeichnet, daß die bewegliche Wand einen den Überströmspalt in zwei Teilspalte aufteilenden Strömungsteiler in Form einer beidseitig überströmbaren, gegebenenfalls dehnbaren, Folie bildet, welche zwischen den Überströmspalt limitierenden Wänden der Filterelemente (PM) beweglich ist.

21. Filtermodul nach Anspruch 4 und 8, dadurch gekennzeichnet, daß die Überströmkammer rechteckig ausgebildet ist und eine rechteckige Folie (FM) nur an dem der Anströmseite zugewandten Ende als Strömungsteiler im Gehäuse eingespannt ist und mit ihrem anderen Ende frei beweglich im Überströmspalt fahnenartig flattert.

22. Filtermodul nach Anspruch 4,8 und 20, dadurch gekennzeichnet, daß die Überströmkammer rechteckig ausgebildet ist und eine rechteckige Folie (FM) nur an den der Anströmseite und der Abströmseite zugewandten Enden als Strömungsteiler im Gehäuse eingespannt ist, wobei die Folienlänge zwischen den Einspannstellen etwas größer gehalten ist als die Distanz zwischen den Einspannstellen.

23. Vorrichtung nach Anspruch 4 und 14, gekennzeichnet durch die Zuordnung einer Steuereinrichtung (9...22) für die aufeinander abgestimmte Führung der Flüssigkeitsströme und Spaltverengung und Spaltvergrößerung.

24. Vorrichtung nach Anspruch 4 und 14, gekennzeichnet durch die Zuordnung einer Steuereinrichtung (9...22) für die aufeinander abgestimmte Führung der Flüssigkeitsströme und Spaltverengung und Spaltvergrößerung sowie zur Entnahme von Filtratproben aus einem geschlossenen Flüssigkeitskreislauf, insbesondere Fermenterkreislauf.

**Fig. 1**

**Fig. 2**

**Fig. 3**

**Fig. 4**

Fig. 5

Fig. 6

Fig. 7

$\underline{\textit{Fig. 8}}$

EP 0 341 573 A2

**Fig. 9**

Fig. 10

Fig. 11

*Fig. 12*

EP 0 341 573 A2